(19) Europäisches Patentamt
European Patent Office
Office européen des brevets

(11) **EP 2 358 430 B1**

(12) **EUROPÄISCHE PATENTSCHRIFT**

(45) Veröffentlichungstag und Bekanntmachung des Hinweises auf die Patenterteilung:
**23.01.2013  Patentblatt 2013/04**

(21) Anmeldenummer: 09741225.8

(22) Anmeldetag: **16.10.2009**

(51) Int Cl.:
*A61N 1/36* (2006.01)     *A61N 1/05* (2006.01)

(86) Internationale Anmeldenummer:
**PCT/EP2009/007452**

(87) Internationale Veröffentlichungsnummer:
**WO 2010/043413 (22.04.2010 Gazette 2010/16)**

(54) **VORRICHTUNG ZUR KONDITIONIERTEN DESYNCHRONISIERENDEN STIMULATION**

DEVICE FOR CONDITIONED DESYNCHRONIZED STIMULATION

DISPOSITIF DE STIMULATION CONDITIONNELLE DE DESYNCHRONISATION

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **17.10.2008   DE 102008052078**

(43) Veröffentlichungstag der Anmeldung:
**24.08.2011   Patentblatt 2011/34**

(73) Patentinhaber: **Forschungszentrum Jülich GmbH 52425 Jülich (DE)**

(72) Erfinder: **TASS, Peter 81541 München (DE)**

(74) Vertreter: **Manitz, Finsterwald & Partner GbR Martin-Greif-Strasse 1 80336 München (DE)**

(56) Entgegenhaltungen:
**EP-A2- 1 944 059     DE-A1- 10 233 960**

**Beschreibung**

Die Erfindung betrifft eine Vorrichtung zur konditionierten desynchronisierenden Stimulation.

Bei Patienten mit neurologischen oder psychiatrischen Erkrankungen, z.B. Morbus Parkinson, essentiellem Tremor, Dystonie oder Zwangserkrankungen, sind Nervenzellverbände in umschriebenen Bereichen des Gehirns, z.B. des Thalamus und der Basalganglien, krankhaft, z.B. übersteigert synchron, aktiv. In diesem Fall bildet eine große Anzahl von Neuronen synchron Aktionspotentiale aus, d.h. die beteiligten Neuronen feuern übermäßig synchron. Beim Gesunden hingegen feuern die Neuronen in diesen Hirngebieten qualitativ anders, z.B. auf unkorrelierte Weise.

Beim Morbus Parkinson verändert die pathologisch synchrone Aktivität die neuronale Aktivität in anderen Hirngebieten, z.B. in Arealen der Großhirnrinde wie dem primär motorischen Cortex. Dabei zwingt die pathologisch synchrone Aktivität im Bereich des Thalamus und der Basalganglien beispielsweise den Großhirnrindenarealen ihren Rhythmus auf, sodass schließlich die von diesen Arealen gesteuerten Muskeln pathologische Aktivität, z.B. ein rhythmisches Zittern (Tremor), entfalten.

Neurologische und psychiatrische Erkrankungen mit übermäßig stark ausgeprägter neuronaler Synchronisation werden bis jetzt - bei Versagen einer medikamentösen Therapie - durch elektrische Hirnstimulation behandelt.

In der Schrift EP 1 944 059 A2 ist eine Stimulationseinheit zur Erzeugung von elektrischen Reizen beschrieben, die bei einer Verabreichung an das Gehirn und/oder das Rückenmark eines Patienten eine krankhaft synchrone Aktivität von Neuronen im Gehirn und/oder Rückenmark des Patienten unterdrücken. Aus der Schrift DE 102 33 960 A1 sind Vorrichtungen bekannt, die - zum gleichen Zweck - optische, akustische, taktile oder vibratorische Reize erzeugen.

Vor diesem Hintergrund wird eine Vorrichtung gemäß Anspruch 1 angegeben. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1          eine schematische Darstellung einer Vorrichtung 100 zur konditionierten desynchronisierenden Stimulation gemäß einem Ausführungsbeispiel während des Betriebs;

Fig. 2A und 2B     schematische Darstellungen von zwei unterschiedlichen Betriebsmodi der Vorrichtung 100;

Fig. 3          eine schematische Darstellung einer Vorrichtung 300 zur konditionierten desynchronisierenden Stimulation gemäß einem weiteren Ausführungsbeispiel während des Betriebs;

Fig. 4          eine schematische Darstellung eines Ausführungsbeispiels einer Stimulationselektrode 14;

Fig. 5          eine schematische Darstellung der Vorrichtung 100 mit der Stimulationselektrode 14 während des Betriebs;

Fig. 6          eine schematische Darstellung von spezifischen elektrischen Reizen, die mittels mehrerer Stimulationskontaktflächen appliziert werden;

Fig. 7          eine schematische Darstellung von Sequenzen von elektrischen Pulszügen, die mittels mehrerer Stimulationskontaktflächen appliziert werden;

Fig. 8          eine schematische Darstellung eines elektrischen Pulszugs;

Fig. 9          eine schematische Darstellung einer Variation der in Fig. 6 gezeigten Stimulation;

Fig. 10          eine schematische Darstellung einer weiteren Variation der in Fig. 6 gezeigten Stimulation;

Fig. 11          eine schematische Darstellung der Vorrichtung 100 zur konditionierten desynchronisierenden Stimulation gemäß einem Ausführungsbeispiel während des Betriebs;

Fig. 12A und 12B   schematische Darstellungen eines Ausführungsbeispiels einer Stimulationseinheit zur Erzeugung und Applikation von unspezifischen optischen, akustischen, taktilen Reizen und Vibrationsreizen; und

Fig. 13          eine schematische Darstellung eines weiteren Ausführungsbeispiels einer Stimulationseinheit zur Erzeugung und Applikation von akustischen Reizen.

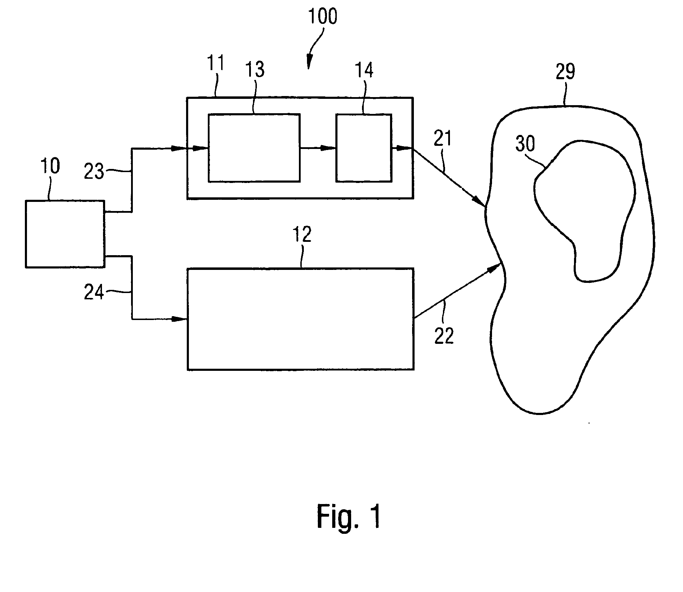

In Fig. 1 ist schematisch eine Vorrichtung 100 zur konditionierten desynchronisierenden Stimulation dargestellt. Die Vorrichtung 100 besteht aus einer Steuereinheit 10, einer ersten Stimulationseinheit 11 und einer zweiten Stimulationseinheit 12. Die erste Stimulationseinheit 11 erzeugt elektrische erste Reize 21 und die zweite Stimulationseinheit erzeugt sensorische zweite Reize 22. Bei den sensorische zweiten Reizen 22 handelt sich um ein oder mehrere Reize aus der Gruppe von optischen, akustischen, taktilen Reizen und Vibrationsreizen. Die Steuereinheit 10 dient zur Steuerung der beiden Stimulationseinheiten 11 und 12 mittels Steuersignalen 23 bzw. 24.

Die Vorrichtung 100 kann insbesondere zur Behandlung von neurologischen oder psychiatrischen Erkrankungen, z.B. Morbus Parkinson, essentiellem Tremor, Dystonie, Epilepsie, Tremor infolge von Multipler Sklerose sowie anderen pathologischen Tremores, Depression, Bewegungsstörungen, Kleinhirnerkrankungen, Zwangserkrankungen, Tourette-Syndrom, Funktionsstörungen nach Schlaganfall, Spastik, Tinnitus, Schlafstörungen, Schizophrenie, Suchterkrankungen, Persönlichkeitsstörungen, Aufmerksamkeits-Defizit-Syndrom, Aufmerksamkeits-Defizit-Hyperaktivitäts-Syndrom, Spielsucht, Neurosen, Fresssucht, Burnout-Syndrom, Fibromyalgie, Migräne, Cluster-Kopfschmerz, allgemeiner Kopfschmerz, Neuralgie, Ataxie, Tic-Störung oder Hypertonie, aber auch anderen Krankheiten verwendet werden.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d.h. die beteiligten Neuronen feuern übermäßig synchron. Hinzu kommt, dass die kranke Neuronenpopulation eine oszillatorische neuronale Aktivität aufweist, d.h. die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z.B. auf unkorrelierte Weise.

In Fig. 1 ist die Vorrichtung 100 während ihres bestimmungsgemäßen Betriebs dargestellt. Während des Betriebs werden die elektrischen ersten Reize 21 an das Gehirn 29 und/oder Rükkenmark 29 eines Patienten appliziert. Mindestens eine Neuronenpopulation 30 im Gehirn 29 oder Rückenmark 29 des Patienten weist eine wie vorstehend beschriebene krankhaft synchrone neuronale Aktivität auf. Zur Erzeugung und Applikation der ersten Reize 21 kann die erste Stimulationseinheit 10 eine Generatoreinheit 13 sowie mindestens eine Stimulationselektrode 14 enthalten. Die Generatoreinheit 13 erzeugt die ersten Reize 21, die mittels geeigneter Verbindungsleitungen in die Stimulationselektrode 14 eingespeist werden. Die Stimulationselektrode 14 ist operativ derart im Gehirn 29 oder im Bereich des Rükkenmarks 29 des Patienten platziert worden, dass die erste Reize 21 der krankhaft aktiven Neuronenpopulation 30 verabreicht werden oder zumindest an Bereiche des Gehirns 29 oder Rückenmarks 29, von wo aus die ersten Reize 21 über das Nervensystem an die krankhaft aktive Neuronenpopulation 30 weitergeleitet werden. Die ersten Reize 21 sind so ausgestaltet, dass sie die krankhaft synchrone Aktivität der Neuronenpopulation 30 unterdrücken oder gar zu einer Desynchronisation der Neuronenpopulation 30 führen. Da es sich bei den ersten Reizen 21 um therapeutisch wirksame elektrische Reize handelt, werden sie auch als "spezifische" Reize bezeichnet.

Die zweite Stimulationseinheit 12 erzeugt optische, akustische, taktile und/oder vibratorische zweite Reize 22. Die optischen bzw. akustischen zweiten Reize 22 werden über die Augen bzw. die Ohren des Patienten aufgenommen und an das Nervensystem weitergeleitet. Die taktilen zweiten Reizen 22 können Druck- und/oder Berührungsreize sein, die von in der Haut des Patienten gelegenen Rezeptoren aufgenommen werden. Zu diesen Rezeptoren zählen insbesondere Merkel-Zellen und Ruffini-Körperchen, die als Druckrezeptoren und insbesondere Intensitätsdetektoren wirken, sowie Meissner-Körperchen und Haarfollikelrezeptoren, die als Berührungsrezeptoren und insbesondere Geschwindigkeitsdetektoren wirken. Im Unterschied zu den sich auf die Oberflächensensibilität der Haut beziehenden taktilen zweiten Reize 22 zielen die vibratorischen zweiten Reize 22 vorwiegend auf die Tiefensensibilität ab. Die vibratorischen zweiten Reize 22 können von in der Haut, den Muskeln, dem Subkutangewebe und/oder den Sehnen des Patienten gelegenen Rezeptoren aufgenommen werden. Als Rezeptoren für die vibratorischen zweiten Reize 22 seien beispielhaft die Vater-Pacini-Körperchen genannt, die Vibrationsempfindungen und Beschleunigungen vermitteln.

Die zweiten Reize 22 sind von dem Patienten bewusst wahrnehmbar und für den Patienten insbesondere nicht unangenehm. Die sensorischen zweiten Reize 22 haben für sich alleine angewandt, d.h. ohne das weiter unten beschriebene Zusammenwirken mit den ersten Reizen 21 in der Lernphase, keine oder kaum eine desynchronisierende oder Koinzidenzraten-senkende Wirkung auf die krankhaft synchrone neuronale Aktivität der Neuronenpopulation 30. Die von der zweiten Stimulationseinheit 12 applizierten zweiten Reize 22 werden daher auch als "unspezifische" Reize bezeichnet.

Zur Applikation der ersten und zweiten Reize 21 und 22 kann die Vorrichtung 100 in zwei verschiedenen Betriebsmodi betrieben werden. Der jeweilige Betriebsmodus kann beispielsweise vorgegeben sein oder kann von der Steuereinheit 10 ausgewählt werden. Die Steuereinheit 10 steuert die beiden Stimulationseinheiten 11 und 12 entsprechend dem ausgewählten Betriebsmodus an.

In einem ersten Betriebsmodus, der auch als Lernphase bezeichnet wird, werden die unspezifischen zweiten Reize 22 zumindest teilweise zeitlich eng gekoppelt an die Applikation der spezifischen ersten Reize 21 dem Patienten verabreicht, d.h. die ersten und zweiten Reize 21 und 22 werden im ersten Betriebsmodus zumindest teilweise in Paaren verabreicht. Das Nervensystem des Patienten wird hierdurch konditioniert, d.h. es lernt, auf die unspezifischen zweiten Reize 22 so zu reagieren wie auf die spezifischen ersten Reize 21, auch wenn die spezifischen ersten Reize 21 nicht appliziert werden. Dies wird ausgenutzt, indem in dem zweiten Betriebsmodus, der eigentlichen Stimulationsphase, die ersten und zweiten Reize 21 und 22 nicht immer gepaart verabreicht werden; vielmehr werden zwischen solchen Paaren aus ersten und zweiten Reizen 21 und 22 auch unspezifische zweite Reize 22 alleine appliziert. Da die zweiten Reize 22 durch die im ersten Betriebsmodus, d.h. der Lernphase, erzielte Konditionierung des Nervensystems des Patienten auch therapeutische Effekte erzielen, wird der für die Therapie notwendige Stromeintrag in das Gewebe des Patienten im zweiten Betriebsmodus, also in der eigentlichen Stimulationsphase, gesenkt. Gegenüber einer herkömmlichen elektrischen Hirnstimulation kann mit der Vorrichtung 100 der Stromeintrag z.B. bis um den Faktor 10 und mehr vermindert werden.

Durch den deutlich gesunkenen Stromeintrag ergibt sich der Vorteil, dass die Wahrscheinlichkeit, mit welcher Nebenwirkungen auftreten, deutlich reduziert wird. Je mehr Stimulationsstrom nämlich appliziert wird, desto größer ist die Wahrscheinlichkeit, dass nicht nur das Zielareal, sondern auch benachbarte Areale mit stimuliert werden. Dies führt zu einer Reihe von dem Fachmann bekannten, für den Patienten zum Teil erheblich unangenehmen Nebenwirkungen.

Ein weiterer Vorteil des gesunkenen Stromeintrags gegenüber herkömmlichen Stimulationsverfahren ist, dass mit dem verringerten Stromeintrag zu Stimulationszwecken ein deutlich geringerer Energiebedarf der Generatoreinheit einhergeht, die üblicherweise in den Patienten implantiert wird. Da die Batteriegröße maßgeblich die Größe der Generatoreinheit bestimmt, kann die Generatoreinheit somit kleiner ausgelegt werden. Dies ist für den Patienten u.a. aus kosmetischen Gründen deutlich angenehmer. Außerdem wird das Infektionsrisiko in der Generatortasche, welches mit der Größe der Generatoreinheit korreliert, gesenkt. Je nach Größe der Generatoreinheit beträgt das Infektionsrisiko bei zur Zeit verwandten Generatoreinheiten ca. 5%, d.h. bei 5% der Patienten kommt es nach der Implantation der Generatoreinheit zu einer Infektion in der Generatortasche, d.h. dem Gewebe, welches die Generatoreinheit umschließt. Durch die deutliche Verringerung des Stromeintrags kann schließlich die Generatorgröße z.B. so minimiert werden, dass die gesamte Generatoreinheit in dem in den Schädelknochen eingebrachten Bohrloch platziert werden kann.

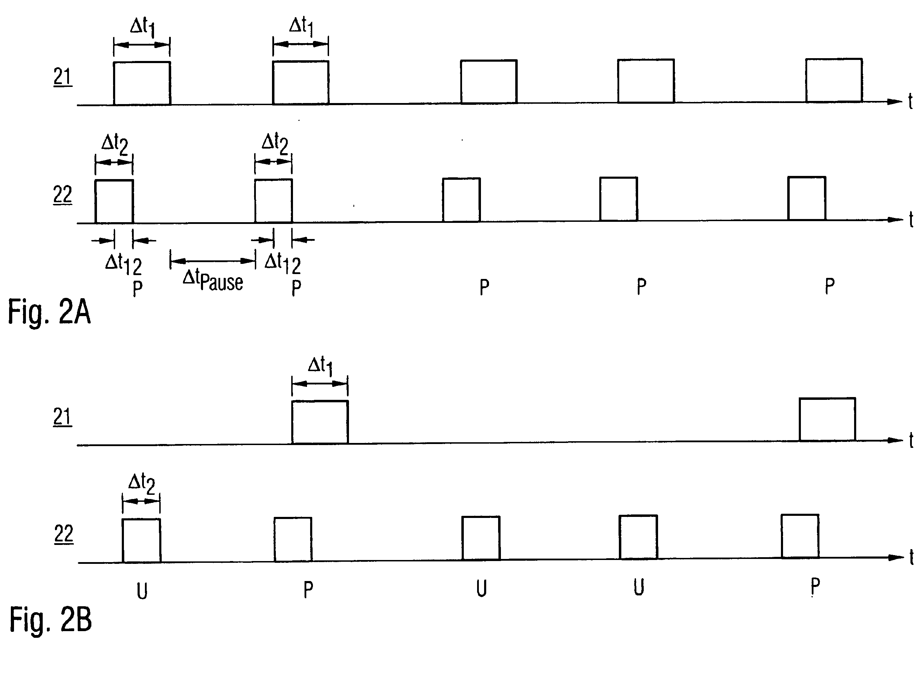

In den Fig. 2A und 2B sind beispielhaft die Unterschiede bei der Applikation der ersten und zweiten Reize 21 und 22 in dem ersten und dem zweiten Betriebsmodus graphisch dargestellt. In Fig. 2A sind untereinander erste Zeitabschnitte $\Delta t_1$ und zweite Zeitabschnitte $\Delta t_2$ gegen die Zeit t aufgetragen, während derer die ersten Reize 21 bzw. die zweite Reize 22 in dem ersten Betriebsmodus erzeugt und dem Patienten verabreicht werden. Die Zeitabschnitte $\Delta t_1$ und $\Delta t_2$ sind jeweils durch Rechtecke dargestellt. Aus Fig. 2A geht hervor, dass in dem ersten Betriebsmodus die Erzeugung und Applikation der unspezifischen zweiten Reize 22 an die Erzeugung und Applikation der spezifischen ersten Reize 21 gekoppelt ist. Die Zeitabschnitte $\Delta t_1$ und $\Delta t_2$ treten in der Lernphase paarweise auf. Durch die gepaarte Applikation der Reize 21 und 22 wird das Gehirn 29 und/oder Rückenmark 29 des Patienten konditioniert, d.h. nach der Lernphase (z.B. schon nach zwei oder mehr gepaarten Zeitabschnitten $\Delta t_1$ und $\Delta t_2$) ruft auch ein unspezifischer zweiter Reiz 22, der ohne einen zusätzlichen spezifischen ersten Reiz 21 appliziert wird, eine therapeutische Wirkung wie ein spezifischer erster Reiz 21 hervor. Vor dieser Lernphase hätte ein unspezifischer zweiter Reiz 22 keine therapeutische Wirkung hervorgerufen.

Die Dauer der Zeitabschnitte $\Delta t_1$, in denen die spezifischen ersten Reize 21 appliziert werden, beträgt z.B. zwischen 30 Minuten und 6 Stunden, kann aber auch außerhalb dieses Bereichs liegen. Die Dauer der Zeitabschnitte $\Delta t_2$, in denen die unspezifischen zweiten Reize 22 appliziert werden, beträgt z.B. zwischen 10 Minuten und 6 Stunden, kann aber auch außerhalb dieses Bereichs liegen. Beispielsweise überlappen in dem ersten Betriebsmodus die Zeitabschnitte $\Delta t_1$ mit den jeweils zugehörigen Zeitabschnitten $\Delta t_2$. Dieser Überlapp $\Delta t_{12}$ beträgt z.B. mindestens 10% oder 20% oder 30% oder 40% oder 50% oder 60% oder 70% oder 80% oder mindestens 90% oder gar 100% des jeweiligen Zeitabschnitts $\Delta t_2$. Bei einander zugeordneten Zeitabschnitten $\Delta t_1$ und $\Delta t_2$ kann wie in Fig. 2A dargestellt der Zeitabschnitt $\Delta t_2$ zuerst beginnen, es ist alternativ aber auch möglich, dass mit dem Zeitabschnitt $\Delta t_1$ begonnen wird. Zwischen aufeinander folgenden Paaren von ersten und zweiten Reizen 21 und 22 werden Pausen eingehalten, deren Länge $\Delta t_{pause}$ z.B. zwischen 3 Stunden und 24 Stunden betragen kann. Sowohl die Längen der Zeitabschnitte $\Delta t_1$ und $\Delta t_2$ als auch die Überlappzeiträume $\Delta t_{12}$ sowie die Stimulationspausen $\Delta t_{pause}$ können während einer Stimulationsphase variiert werden. Die Dauer der Lernphase, d.h. die Dauer, in der die Vorrichtung im ersten Betriebsmodus betrieben wird, kann vorgegeben sein und kann beispielsweise eine vorgegebene Anzahl von gepaarten Zeitabschnitten $\Delta t_1$ und $\Delta t_2$ umfassen.

Im Folgenden werden Beispiele für die Applikation der ersten und zweiten Reize 21 und 22 während der Lernphase beschrieben. Gemäß einem Beispiel können während eines Zeitabschnitts $\Delta t_1$ von 6 Stunden und eines Zeitabschnitts $\Delta t_2$ von 6,25 Stunden erste Reize 21 bzw. zweite Reize 22 appliziert werden, wobei der Zeitabschnitt $\Delta t_2$ 15 Minuten vor dem Zeitabschnitt $\Delta t_1$ beginnt und beide Zeitabschnitte $\Delta t_1$ und $\Delta t_2$ somit gleichzeitig enden. Nach einer Pause $\Delta t_{pause}$ von z.B. 6 Stunden könnte dieser Vorgang wiederholt werden. Um ein zügiges Erlernen bzw. Konditionieren des Nervensystems zu erzielen, könnte die Anzahl der Lernereignisse, d.h. der gepaarten Verabreichung von ersten und zweiten Reizen 21 und 22, gegenüber dem vorstehenden Beispiel noch weiter erhöht werden. So könnten die

Zeitabschnitte $\Delta t_1$ und $\Delta t_2$ z.B. auf 3 bzw. 3,125 Stunden reduziert werden, wobei der Zeitabschnitt $\Delta t_2$ 7,5 Minuten vor dem Zeitabschnitt $\Delta t_1$ beginnt. Nach einer Pause $\Delta t_{-Pause}$ von z.B. 3 Stunden könnte die gekoppelte Stimulation erneut durchgeführt werden.

Ein Lerneffekt kann sich eventuell schon nach zwei Applikationen von miteinander gekoppelten ersten und zweiten Reizen 21 und 22 einstellen. Um die Konditionierung des Nervensystems möglichst robust zu gestalten und die Konditionierung in der eigentlichen Stimulationsphase möglichst lange ausnutzen zu können, können z.B. 10 bis 50 Paarapplikationen in der Lernphase, d.h. dem ersten Betriebsmodus, durchgeführt werden.

Während der Lernphase muss nicht notwendigerweise jeder Zeitabschnitt $\Delta t_2$ einem der Zeitabschnitte $\Delta t_1$ zugeordnet sein. Beispielsweise kann nach einer bestimmten Anzahl von miteinander gekoppelten Zeitabschnitten $\Delta t_1$ und $\Delta t_2$ ein Zeitabschnitt $\Delta t_1$ oder $\Delta t_2$ eingefügt werden, der nicht an einen zugehörigen Zeitabschnitt $\Delta t_2$ bzw. $\Delta t_1$ gekoppelt ist und währenddessen lediglich erste Reize 21 bzw. zweite Reize 22 erzeugt und appliziert werden. Beispielsweise können im ersten Betriebsmodus mindestens 50% oder 60% oder 70% oder 80% oder 90% oder gar 100% der Zeitabschnitte $\Delta t_2$ an einen zugehörigen Zeitabschnitt $\Delta t_1$ gekoppelt sein. Ferner können im ersten Betriebsmodus mindestens 50% oder 60% oder 70% oder 80% oder 90% oder gar 100% der Zeitabschnitte $\Delta t_1$ an einen zugehörigen Zeitabschnitt $\Delta t_2$ gekoppelt sein.

Anschließend an die im ersten Betriebsmodus durchgeführte Lernphase erfolgt die eigentliche Stimulationsphase. Dazu schaltet die Steuereinheit 10 in den zweiten Betriebsmodus. Beispielhaft sind in Fig. 2B untereinander die Zeitabschnitte $\Delta t_1$ und $\Delta t_2$ gegen die Zeit t aufgetragen, während derer die ersten Reize 21 bzw. die zweite Reize 22 im zweiten Betriebsmodus erzeugt und appliziert werden.

In der eigentlichen Stimulationsphase wird der Umstand ausgenutzt, dass unspezifische zweite Reize 22 aufgrund der in der Lernphase erzielten Konditionierung des Nervensystems des Patienten auch therapeutische Wirkung haben. Hierzu werden - anders als in der Lernphase - nicht hauptsächlich Paare, bestehend aus ersten und zweiten Reizen 21 und 22, appliziert, vielmehr werden auch immer wieder während eines Zeitabschnitts $\Delta t_2$ nur zweite Reize 22 appliziert, die nicht an die Applikation eines ersten Reizes 21 gekoppelt sind. Beispielsweise ist im zweiten Betriebsmodus mindestens 10% oder 20% oder 30% oder 40% oder 50% oder 60% oder 70% oder 80% oder 90% der Zeitabschnitte $\Delta t_2$ kein Zeitabschnitt $\Delta t_1$ zugeordnet, d.h. insgesamt ist die Zahl der Zeitabschnitte $\Delta t_1$ im zweiten Betriebsmodus kleiner als die Zahl der zweiten Zeitabschnitte $\Delta t_2$. Vereinzelt können im zweiten Betriebsmodus gemäß einer Ausführung auch Zeitabschnitte $\Delta t_1$ eingefügt werden, die nicht an einen Zeitabschnitt $\Delta t_2$ gekoppelt sind. Gemäß einer weiteren Ausführung kann z.B. auch vorgesehen sein, dass im zweiten Betriebsmodus sämtlichen Zeitabschnitten $\Delta t_2$ kein Zeitabschnitt $\Delta t_1$ zugeordnet ist.

Die Paare "P" bestehend aus spezifischen und unspezifischen Reizen 21 und 22 und die allein applizierten unspezifischen Reize "U" können im zweiten Betriebsmodus z.B. in periodischen Abfolgen verabreicht werden, z.B. in folgender Reihenfolge: P-P-U-U-U-P-P-U-U-U-P-P-U-U-U-... Das zeitliche Muster, nach dem die unspezifischen zweiten Reize 22 alleine auftreten, kann aber auch deterministisch oder stochastisch oder gemischt deterministisch-stochastisch sein, z.B. kann folgende Reihenfolge gewählt werden: P-P-U-U-U-P-P-U-U-U-U-U-P-P-U-U-U-P-P-U-U-P-P-U-U-U-U-U-P-P-U-U-U-...

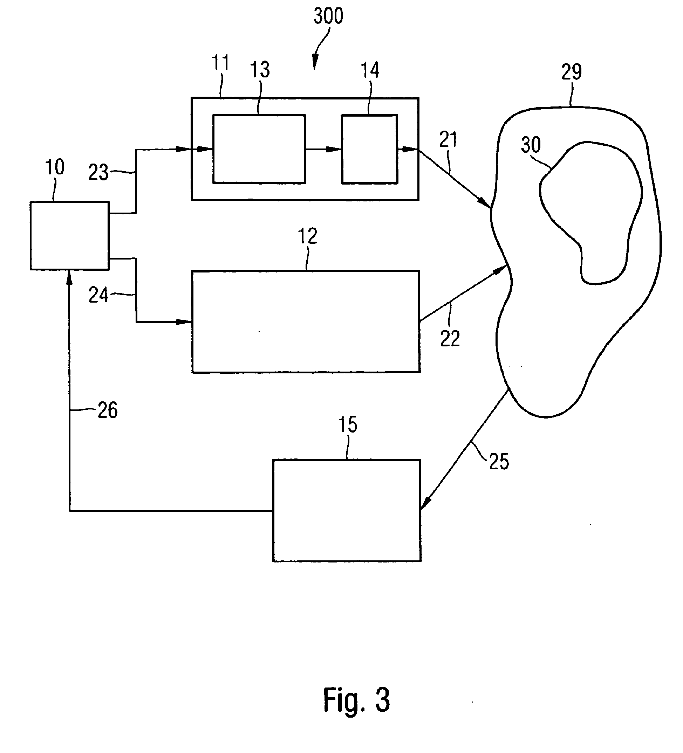

Der mittels der Vorrichtung 100 erzielte Stimulationseffekt kann beispielsweise mit Hilfe einer Messeinheit kontrolliert werden. Eine Vorrichtung 300, die eine solche Messeinheit 15 enthält, ist schematisch in Fig. 3 dargestellt. Die übrigen Komponenten der Vorrichtung 300 sind identisch zu denen der in Fig. 1 gezeigten Vorrichtung 100. Die Messeinheit 15 nimmt ein oder mehrere am Patienten gemessene Messsignale 25 auf, wandelt diese gegebenenfalls in elektrische Signale 26 um und führt diese der Steuereinheit 10 zu. Insbesondere kann mittels der Messeinheit 15 die neuronale Aktivität in dem stimulierten Zielgebiet, d.h. z.B. die neuronale Aktivität der in Fig. 3 schematisch dargestellten Neuronenpopulation 30, oder einem mit der Neuronenpopulation 30 verbundenen Gebiet gemessen werden.

Die Messeinheit 15 kann in Form eines oder mehrerer Sensoren in den Körper des Patienten implantiert sein. Als invasive Sensoren können beispielsweise Tiefenhirnelektroden, sub- oder epidurale Hirnelektroden, subkutane EEG-Elektroden und sub- oder epidurale Rückenmarkselektroden dienen. Des Weiteren können an peripheren Nerven zu befestigende Elektrode als Sensoren eingesetzt werden. Der invasive Sensor kann beispielsweise dieselbe Elektrode 14 sein, die auch zur Applikation der ersten Reize 21 eingesetzt wird.

Die Messsignale 25 können in den Pausen zwischen der Verabreichung der spezifischen ersten Reize 21, aber insbesondere auch während der ausschließlichen Verabreichung der unspezifischen zweiten Reize 22 aufgenommen werden. Sofern die neuronale Aktivität der Zielpopulation 30 gemessen wird, kann die Amplitude der krankhaften Schwingungen in typischen Frequenzbereichen der lokalen Feldpotentiale ermittelt werden, also z.B. bei akinetischen Parkinsonpatienten die integrale Leistung im Beta-Frequenzbereich zwischen 10 und 30 Hz. Bei einer effektiven Stimulation sinkt diese Amplitude ab. Lässt die Stimulationswirkung der allein applizierten unspezifischen zweiten Reize 22 im zweiten Betriebsmodus nach und übersteigt die gemessene Amplitude einen vorgegebenen Schwellwert, so kann die nächste Lernphase im ersten Betriebsmodus erfolgen. Danach kann wieder die eigentliche Stimulation im zweiten Betriebsmodus durchgeführt werden.

Der Schwellwert kann vom Arzt individuell für den jeweiligen Patienten eingestellt werden. Alternativ können typische Werte als Voreinstellung für den Schwellwert gewählt werden, z.B. der Mittelwert der Amplitude plus zweimal die Standardabweichung in Bereichen des Frequenzspektrums ohne Frequenzpeaks und oberhalb von z.B. 70 Hz.

Zusätzlich zu den invasiven Sensoren oder alternativ dazu können auch ein oder mehrere nicht-invasive Sensoren eingesetzt werden, wie z.B. Elektroenzephalographie (EEG)-Elektroden, Magnetenzephalographie (MEG)-Sensoren und Elektromyographie (EMG)-Elektroden. Ferner kann z.B. über ein Akzelerometer die krankhaft oszillatorische Aktivität im Tremor-Frequenzbereich oder die Bewegungsarmut (im Sinne einer Verminderung der Gesamtbewegungen) gemessen werden. Wird ein vorgegebener Wert der Tremoraktivität überschritten bzw. ein kritischer Wert der mittleren stündlichen Aktivität (außerhalb der Nachtzeiten) unterschritten, so beginnt z.B. die nächste Lernphase im ersten Betriebsmodus.

Es ist auch denkbar, zwei Schwellwerte für die Steuerung der beiden Betriebsmodi zu verwenden. Beispielsweise können zwei Schwellwerte $A_L$ und $A_S$ vorgegeben werden, mit denen die von der Messeinheit 15 gemessene Amplitude der krankhaften neuronalen Aktivität der Neuronenpopulation 30 verglichen wird. Der Schwellwert $A_L$ kann größer als der Schwellwert $A_S$ sein und den gröberen der beiden Schwellwerte darstellen. Übersteigt die Amplitude der Betaband-Aktivität den Wert $A_L$, so wird von dem zweiten Betriebsmodus in den ersten Betriebsmodus geschaltet und eine erneute Lernphase durchgeführt.

Falls die Amplitude der Betaband-Aktivität den feineren Schwellwert $A_S$ während des zweiten Betriebsmodus übersteigt, so wird nicht in den ersten Betriebsmodus geschaltet, sondern die Vorrichtung 300 verbleibt in der eigentlichen Stimulationsphase, es werden jedoch vermehrt Paare "P" aus spezifischen ersten Reizen 21 und unspezifischen zweiten Reizen 22 appliziert. Dazu kann beispielsweise eine nur aus unspezifischen Reizen "U" bestehende Teilfolge (-U-U-U-U-U-) übersprungen werden und es wird zu dem in der Sequenz nächsten Abschnitt gesprungen, der Paare "P" aus spezifischen und unspezifischen Reizen 21 und 22 aufweist. Sofern in dem zweiten Betriebsmodus beispielsweise vorgegeben ist, dass ein bestimmter Prozentsatz der zweiten Reize 22 zusammen mit ersten Reizen 21 appliziert wird, so kann dieser Prozentsatz der Paare "P" beim Überschreiten des Schwellwerts $A_S$ um eine bestimmte Prozentzahl erhöht werden. Als Beispiel sei angenommen, dass im zweiten Betriebsmodus 30% der zweiten Reize 22 als Paare "P" zusammen mit ersten Reize 21 appliziert werden. Beim Überschreiten des Schwellwerts $A_S$ kann dieser Prozentsatz beispielsweise um 20% auf 50% erhöht werden. Sobald die Amplitude der Betaband-Aktivität danach wieder einen weiteren vorgegebenen Schwellwert unterschreitet, kann wieder zu den im zweiten Betriebsmodus beispielhaft vorgesehenen 30% zurückgekehrt werden.

Der Übergang vom zweiten in den ersten Betriebsmodus kann auch vom Patienten durch ein externes Patienten-Programmiergerät gesteuert werden. D.h. der Patient hat die Möglichkeit, an einem kleinen, handlichen externen Gerät eine Taste zu drücken, wenn er sich nicht ausreichend therapiert fühlt, wenn also z.B. sein Tremor oder seine Unbeweglichkeit zu stark sind. Nach einem vordefinierten Modus schaltet dann die Steuereinheit 10 vom zweiten Betriebsmodus in den ersten Betriebsmodus, also in eine erneute Lernphase. Der vordefinierte Modus bedeutet hier, dass dieses Umschalten in den ersten Betriebsmodus schon durch den ersten Tastendruck des Patienten ausgelöst wird. Die Vorrichtung 100 bzw. 300 kann aber auch vom Arzt so eingestellt werden, dass erst nach einer kleineren Anzahl von derartigen Tastendrucken pro vorgegebenem Zeitintervall, z.B. nach 3 Tastendrucken pro halber Stunde, das Umschalten in den ersten Betriebsmodus erfolgt.

Zur Therapiekontrolle registriert die Vorrichtung 100 bzw. 300 die Anzahl und die Zeitpunkte der Tastendrucke. Diese Information kann vom Arzt mittels eines für den Arzt bestimmten externen Programmiergeräts ausgelesen werden.

Weiterhin kann vorgesehen sein, dass nach einer vorgegebenen Zeitdauer vom zweiten Betriebsmodus wieder in den ersten Betriebsmodus, also in die Lernphase, gewechselt wird. Für diesen Umschaltmodus ist nicht notwendigerweise eine Therapiekontrolle mit Hilfe der Messeinheit 15 erforderlich, d.h. dieser Umschaltmodus kann sowohl in der Vorrichtung 100 als auch in der Vorrichtung 300 implementiert sein.

Zur Erzeugung der unspezifischen zweiten Reize 22 kann die zweite Stimulationseinheit 12 z.B. einen Lautsprecher, eine Lichtquelle (bzw. Bildquelle) und/oder einen Vibrator enthalten. Generell sollen die zweiten Reize 22 stark genug sein, damit sie vom Patienten bewusst wahrgenommen werden. Sie sollen aber beispielsweise weder als unangenehm stark noch störend oder gar ablenkend empfunden werden. Als akustische zweite Reize 22 kommen beispielsweise ein Summton, ein Brummton oder eine Melodie infrage, die während der Zeitabschnitte $\Delta t_2$ von dem Lautsprecher erzeugt werden. Sofern optische Signale als zweite Reize 22 eingesetzt werden sollen, können diese z.B. abstrakte oder gegenständliche Muster sein, die während der Zeitabschnitte $\Delta t_2$ entweder statisch sind oder sich zeitlich verändern, z.B. eine Blüte, die sich im Wind bewegt, ein Fisch, der im Wasser schwimmt, ein Vogel, der fliegt, eine Sonne, die aufgeht, usw. Taktile Reize bzw. Vibrationsreize können Vibrationen mit für den Patienten wahrnehmbaren Frequenzen sein, die während der Zeitabschnitte $\Delta t_2$ von einem mechanischen Vibrator erzeugt werden. Wahrnehmbare Vibrationsreize können Frequenzen im Bereich von 10 bis 160 Hz oder auch darüber aufweisen, während taktile Reize deutlich geringere Frequenzen haben, die z.B. kleiner als 1 Hz sind. Es können auch Mischformen von taktilen und vibratorischen Reizen eingesetzt werden. Die taktilen bzw. vibratorischen Reize können z.B. von dem Patienten selbst als angenehm ausgewählt werden. Mittels des Vibrators kann ferner während der Zeitabschnitte $\Delta t_2$ auf die Haut des Patienten eine leichte, angenehm massierende Wirkung ausgeübt werden.

Die unspezifischen zweiten Reize 22 können vom Anfang bis zum Ende eines jeweiligen Zeitabschnitts $\Delta t_2$ dem Patienten kontinuierlich verabreicht werden. Alternativ können auch Applikationspausen während der Zeitabschnitte $\Delta t_2$ eingehalten werden, beispielsweise können die zweiten Reize 22 in bestimmten Zeitintervallen mit dazwischen liegenden Applikationspausen während der Zeitabschnitte $\Delta t_2$ verabreicht werden. Diese Zeitmuster können auch variiert werden, z.B. stochastisch oder deterministisch oder gemischt stochastisch-deterministisch. Es kann vorgesehen sein, dass während mindestens 60% oder 70% oder 80% oder 90% der Zeitdauer eines jeweiligen Zeitabschnitts $\Delta t_2$ die zweiten Reize 22 appliziert werden.

Als spezifische erste Reize 21 werden desynchronisierende elektrische Stimulationssignale verwendet oder elektrische Stimulationssignale, die zumindest eine Senkung der Koinzidenzrate der kranken Neuronen bewirken. Die Stimulationselektrode 14, mittels welcher die ersten Reize 21 auf das Gehirn 29 oder Rückenmark 29 des Patienten übertragen werden, kann beispielsweise eine oder zwei oder mehrere Stimulationskontaktflächen aufweisen, die nach der Implantation mit dem Gewebe des Gehirns 29 oder Rückenmarks 29 in Kontakt stehen und über die die elektrischen ersten Reize 21 appliziert werden.

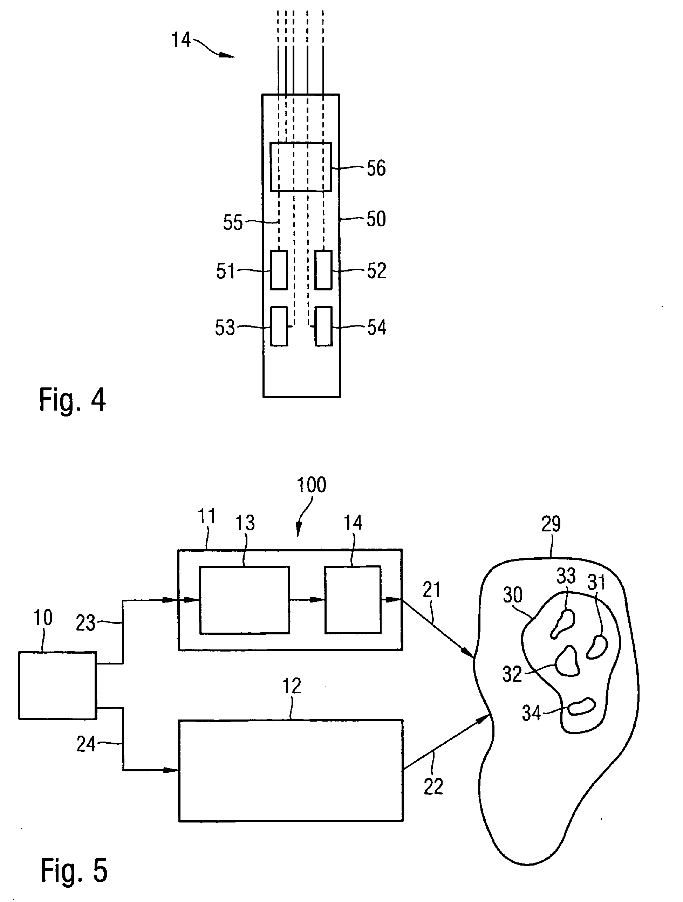

In Fig. 4 ist schematisch und beispielhaft eine Stimulationselektrode 14 dargestellt. Die Stimulationselektrode 14 besteht aus einem isolierten Elektrodenschaft 50 und mindestens einer, beispielsweise zwei oder mehr Stimulationskontaktflächen, die in den Elektrodenschaft 50 eingebracht worden sind. In dem vorliegenden Beispiel enthält die Stimulationselektrode 14 vier Stimulationskontaktflächen 51, 52, 53 und 54. Der Elektrodenschaft 50 und die Stimulationskontaktflächen 51 bis 54 können aus einem biokompatiblen Material hergestellt sein. Ferner sind die Stimulationskontaktflächen 51 bis 54 elektrisch leitfähig, beispielsweise sind sie aus einem Metall gefertigt, und befinden sich nach der Implantation in direktem elektrischen Kontakt mit dem Nervengewebe. In dem vorliegenden Ausführungsbeispiel kann jede der Stimulationskontaktflächen 51 bis 54 über eine eigene Zuleitung 55 angesteuert werden bzw. es können über die Zuleitungen 55 die aufgenommenen Messsignale abgeführt werden. Als Alternative können auch zwei oder mehr Stimulationskontaktflächen 51 bis 54 an dieselbe Zuleitung 55 angeschlossen sein.

In Fig. 4 sind die Stimulationskontaktflächen 51 bis 54 in Reihen und Spalten angeordnet. Ferner sind die Stimulationskontaktflächen 51 bis 54 als Rechtecke ausgestaltet. Diese Ausgestaltungen sind lediglich beispielhaft zu verstehen. Alternativ zu diesen Ausgestaltungen kann die Stimulationselektrode 14 eine beliebige Anzahl N (N = 1, 2, 3, 4, 5, 6, 7, 8, 9, 10, 11, 12...) von Stimulationskontaktflächen enthalten, die beliebig zueinander angeordnet sein können und beliebige Formen aufweisen können.

Neben den Stimulationskontaktflächen 51 bis 54 kann die Elektrode 14 eine Referenzelektrode 56 aufweisen, deren Oberfläche größer als die der Stimulationskontaktflächen 51 bis 54 sein kann. Die Referenzelektrode 56 wird bei der Stimulation des Nervengewebes zur Erzeugung eines Referenzpotentials eingesetzt. Alternativ kann auch eine der Stimulationskontaktflächen 51 bis 54 zu diesem Zweck verwendet werden. D.h., es kann entweder unipolar zwischen einer einzelnen Stimulationskontaktfläche 51 bis 54 und der Referenzelektrode 56 (oder dem Gehäuse der Generatoreinheit 13) oder bipolar zwischen verschiedenen Stimulationskontaktflächen 51 bis 54 stimuliert werden.

Neben ihrer Funktion als Stimulationselektrode kann die Elektrode 14 auch als Messeinheit 15 innerhalb der Vorrichtung 300 eingesetzt werden. In diesem Fall werden über mindestens eine der Kontaktflächen 51 bis 54 Messsignale aufgenommen.

Die Stimulationskontaktflächen 51 bis 54 können mit der Generatoreinheit 13 über Kabel oder über telemetrische Verbindungen verbunden sein.

Die Mehrzahl von Stimulationskontaktflächen 51 bis 54 ermöglicht es, unterschiedliche Bereiche der Gehirns 29 oder Rükkenmarks 29 über die einzelnen Stimulationskontaktflächen 51 bis 54 separat zu stimulieren. Zu diesem Zweck kann beispielsweise jede der Stimulationskontaktflächen 51 bis 54 über eine eigene Verbindungsleitung 55 mit der Generatoreinheit 13 verbunden sein. Dies ermöglicht es der Generatoreinheit 13, für jede einzelne der Stimulationskontaktflächen 51 bis 54 bestimmte erste Reize 21 zu erzeugen. Die Stimulationskontaktflächen 51 bis 54 können so in den Patienten implantiert sein, dass die auf das Gewebe applizierten ersten Reize 21 über Nervenleitungen an unterschiedliche Zielgebiete, die im Gehirn 29 und/oder Rückenmark 29 liegen, weitergeleitet werden. Folglich können mittels der Vorrichtung 100 bzw. 300 verschiedene Zielgebiete im Gehirn 29 und/oder Rükkenmark 29 während desselben Stimulationszeitraums $\Delta t_1$ mit eventuell unterschiedlichen und/oder zeitversetzten ersten Reizen 21 stimuliert werden.

Aufgrund der Mehrzahl von Stimulationskontaktflächen 51 bis 54 können nicht nur unterschiedliche Bereiche des Gehirns 29 und/oder Rückenmarks 29 stimuliert werden, sondern es können auch andere Stimulationsformen eingesetzt werden, als dies bei der Verwendung beispielsweise nur einer einzigen Stimulationskontaktfläche möglich wäre. Gemäß einer Ausgestaltung werden der Neuronenpopulation 30, die eine krankhaft synchrone und oszillatorische Aktivität aufweist, mittels der Stimulationselektrode 14 erste Reize 21 verabreicht, welche in der Neuronenpopulation 30 ein Zurücksetzen, einen sogenannten Reset, der Phase der neuronalen Aktivität der stimulierten Neuronen bewirken. Durch das Zurücksetzen wird die Phase der stimulierten Neuronen unabhängig von dem aktuellen Phasenwert auf einen bestimmten Phasenwert, z.B. 0°, gesetzt. Somit wird die Phase der neuronalen Aktivität der krankhaften Neuronenpopulation 30 mittels einer gezielten Stimulation kontrolliert. Ferner ist es aufgrund der Mehrzahl von Stimulationskontaktflächen 51 bis 54 möglich, die krankhafte Neuronenpopulation 30 an unterschiedlichen Stellen zu stimulieren. Dies ermöglicht es, die Phase der neuronalen Aktivität der krankhaften Neuronenpopulation 30 an den unterschiedlichen Stimulationsstellen zu unterschiedlichen Zeitpunkten zurückzusetzen. Im Ergebnis wird dadurch die krankhafte Neuronenpopulation 30, deren Neuronen zuvor synchron und mit gleicher Frequenz und Phase aktiv waren, in mehrere Subpopulationen aufgespalten, die in Fig. 5 schematisch dargestellt sind und mit den Bezugszeichen 31, 32, 33 und 34 gekennzeichnet sind. Innerhalb einer der Subpopulationen 31 bis 34 sind die Neuronen nach einem Zurücksetzen der Phase weiterhin synchron und feuern auch weiterhin mit derselben pathologischen Frequenz, aber jede der Subpopulationen 31 bis 34 weist bezüglich ihrer neuronalen Aktivität die Phase auf, die ihr durch den Stimulationsreiz aufgezwungen wurde. Dies bedeutet, dass die neuronalen Aktivitäten der einzelnen Subpopulationen 31 bis 34 nach dem Zurücksetzen ihrer Phasen weiterhin einen in etwa sinusförmigen Verlauf mit derselben pathologischen Frequenz haben, aber unterschiedliche Phasen.

Beispielsweise können die Stimulationskontaktflächen 51 bis 54 derart auf oder in dem Hirn- oder Rückenmarkgewebe 29 des Patienten platziert sein, dass die von der Stimulationskontaktfläche 51 applizierten ersten Reize 21 die Subpopulation 31 reizen und deren neuronale Phase zurücksetzen und die von der Stimulationskontaktfläche 52 applizierten ersten Reize 21 die Subpopulation 52 reizen und deren neuronale Phase zurücksetzen. Analoges gilt für die Stimulationskontaktfläche 53 bzw. 54 in Bezug auf die Subpopulation 33 bzw. 34.

Bedingt durch die krankhafte Interaktion zwischen den Neuronen ist der durch die Stimulation erzeugte Zustand mit mindestens zwei Subpopulationen instabil, und die gesamte Neuronenpopulation 30 nähert sich schnell einem Zustand kompletter Desynchronisation, in welchem die Neuronen unkorreliert feuern. Der gewünschte Zustand, das heißt die komplette Desynchronisation, ist somit nach der Applikation der Stimulationssignale über die Stimulationselektrode 14 nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation 30 in Subpopulationen 31 bis 34 mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die elektrische Stimulation mit der Vorrichtung 100 bzw. 300 eine Neuorganisation der Konnektivität der gestörten neuronalen Netzwerke erzielt werden, sodass lang anhaltende therapeutische Effekte bewirkt werden können. Der erzielte synaptische Umbau ist von großer Bedeutung für die wirksame Behandlung neurologischer oder psychiatrischer Erkrankungen.

Würden anstelle der Stimulationssignale, mit welchen die Phasen der stimulierten Neuronen kontrolliert werden können, anders ausgestaltete Stimulationssignale eingesetzt, z.B. hochfrequente, kontinuierlich applizierte Hochfrequenzpulszüge, könnten die vorstehend beschriebenen lang anhaltenden therapeutischen Effekte typischerweise nicht erzielt werden, was zur Folge hätte, dass dauerhaft und mit vergleichsweise hohen Stromstärken stimuliert werden müsste. Im Gegensatz dazu wird für die hier beschriebenen Stimulationsformen nur wenig von außen in das Neuronensystem eingebrachte Energie benötigt, um einen therapeutischen Effekt zu erzielen. Aufgrund des vergleichsweise geringen Energieeintrags in den Körper des Patienten und die häufig sehr schnell erzielten Stimulationsergebnisse, können mittels der Vorrichtung 100 bzw. 300 die mit einer elektrischen Nervenstimulation häufig einhergehenden Dysästhesien bzw. Parästhesien (schmerzhafte Missempfindungen) erheblich reduziert werden.

Um durch zeitversetztes Zurücksetzen der Phasen der Subpopulationen 31 bis 34 der krankhaft synchronen Neuronenpopulation 30 eine Desynchronisation der gesamten Neuronenpopulation 30 zu erzielen, kann auf verschiedene Arten vorgegangen werden. Beispielsweise können Stimulationssignale, die ein Zurücksetzen der Phase von Neuronen bewirken, zeitversetzt über die unterschiedlichen Stimulationskontaktflächen 51 bis 54 an das jeweils stimulierte Nervengewebe abgegeben werden. Des Weiteren können die Stimulationssignale z.B. phasenversetzt oder mit unterschiedlicher Polarität appliziert werden, sodass sie im Ergebnis auch zu einem zeitversetzten Zurücksetzen der Phasen der unterschiedlichen Subpopulationen 31 bis 34 führen.

Die Vorrichtung 100 kann beispielsweise in einem sogenannten "open loop"-Modus betrieben werden, bei welchem die Generatoreinheit 13 vorgegebene erste Reize 21 erzeugt und diese über die Stimulationskontaktflächen 51 bis 54 an das Nervengewebe abgegeben werden. Des Weiteren kann die Vorrichtung 100 auch zu der in Fig. 3 gezeigten Vorrichtung 300 weitergebildet werden, welche ein sogenanntes "closed loop"-System darstellt. Die Vorrichtung 300 enthält zusätzlich noch die Messeinheit 15, welche ein oder mehrere am Patienten aufgenommene Messsignale 26 bereitstellt und diese an die Steuereinheit 10 weiterleitet.

Mit Hilfe der Messsignale 26 kann eine bedarfsgesteuerte Stimulation durchgeführt werden. Hierzu detektiert die Steuereinheit 10 anhand der von der Messeinheit 15 aufgenommenen Messsignale 26 das Vorhandensein und/oder die Ausprägung eines oder mehrerer krankhafter Merkmale. Beispielsweise kann - wie bereits oben erläutert wurde - die Amplitude oder der Betrag der neuronalen Aktivität gemessen werden, mit einem oder mehreren vorgegebenen Schwellwerten verglichen werden und je nach Ergebnis des Vergleichs ein bestimmter Betriebsmodus ausgewählt werden. Die Generatoreinheit 13 kann so ausgestaltet sein, dass eine Stimulation oder der erste Betriebsmodus gestartet wird, sobald der vorgegebene Schwellwert überschritten wird. Ferner kann anhand der von der Messeinheit 15 aufgenommenen Messsignale 26 beispielsweise die Stärke der ersten Reize 21 eingestellt werden. Z.B. können ein oder mehrere Schwellwerte vorgegeben werden, und bei einem Überschreiten der Amplitude oder des Betrags der Messsignale 26 über einen bestimmten Schwellwert wird eine bestimmte Stärke der ersten Reize 21 eingestellt.

Des Weiteren kann vorgesehen sein, dass die von der Messeinheit 15 aufgenommenen Messsignale 26 direkt oder gegebenenfalls nach einem oder mehreren Verarbeitungsschritten als erste Reize 21 eingesetzt werden und von der Generatoreinheit 13 in die Stimulationselektrode 14 eingespeist werden. Beispielsweise können die Messsignale 26 verstärkt und gegebenenfalls nach mathematischer Verrechnung (z.B. nach Mischung der Messsignale) mit einer Zeitverzögerung und linearen und/oder nichtlinearen Verrechnungsschritten und Kombinationen prozessiert und in die Stimulationselektrode 14 eingespeist werden. Der Verrechnungsmodus wird hierbei so gewählt, dass der krankhaften neuronalen Aktivität entgegengewirkt wird und das Stimulationssignal mit abnehmender krankhafter neuronaler Aktivität ebenfalls verschwindet oder zumindest deutlich in seiner Stärke reduziert wird.

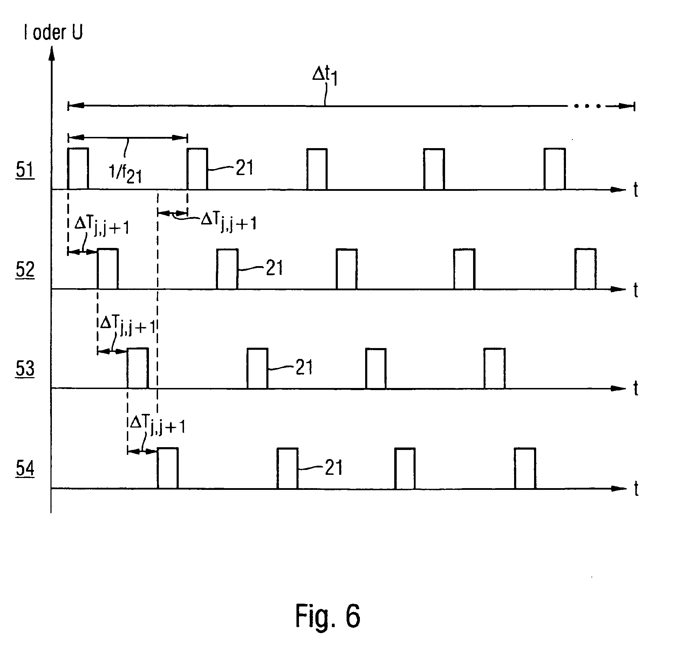

Ein für die oben beschriebenen Zwecke geeignetes Stimulationsverfahren, das beispielsweise mit einer der Vorrichtungen 100 und 300 durchgeführt werden kann, ist in Fig. 6 schematisch dargestellt. Dort sind untereinander die über die Stimulationskontaktflächen 51 bis 54 applizierten ersten Reize 21 gegen die Zeit t aufgetragen. Der in Fig. 6 gezeigte Zeitraum stellt einen Ausschnitt aus einem Zeitabschnitt $\Delta t_1$ dar. Die dargestellte Stimulation kann bis zum Ende des Zeitabschnitts $\Delta t_1$ fortgesetzt werden.

Bei dem in Fig. 6 dargestellten Verfahren verabreicht jede der Stimulationskontaktflächen 51 bis 54 den ersten Reiz 21 periodisch an den jeweiligen Bereich des Gewebes, auf dem die Stimulationskontaktfläche 51 bis 54 platziert ist. Die Frequenz $f_{21}$, mit welcher die ersten Reize 21 pro Stimulationskontaktfläche 51 bis 54 wiederholt werden, kann im Bereich von 1 bis 30 Hz und insbesondere im Bereich von 1 bis 20 Hz oder im Bereich von 5 bis 20 Hz oder im Bereich von 10 bis 30 Hz liegen, kann aber auch kleinere oder größere Werte annehmen.

Gemäß der in Fig. 6 gezeigten Ausgestaltung erfolgt die Verabreichung der ersten Reize 21 über die einzelnen Stimulationskontaktflächen 51 bis 54 mit einer zeitlichen Verzögerung zwischen den einzelnen Stimulationskontaktflächen 51 bis 54. Beispielsweise kann der Beginn zeitlich aufeinander folgender und von unterschiedlichen Stimulationskontaktflächen 51 bis 54 applizierten ersten Reizen 21 um eine Zeit $\Delta T_{j,j+1}$ verschoben sein.

Im Fall von N Stimulationskontaktflächen kann die zeitliche Verzögerung $\Delta T_{j,j+1}$ zwischen jeweils zwei aufeinander folgenden ersten Reizen 21 beispielsweise im Bereich eines N-tels der Periode $1/f_{21}$ liegen. In dem in Fig. 6 gezeigten Ausführungsbeispiel (N = 4) beträgt die Verzögerung $\Delta T_{j,j+1}$ dann $1/(4 \times f_{21})$.

Die Frequenz $f_{21}$ kann beispielsweise im Bereich der mittleren Frequenz der krankhaft rhythmischen Aktivität des Ziel-Netzwerks liegen. Bei neurologischen und psychiatrischen Erkrankungen liegt die mittlere Frequenz typischerweise im Bereich von 1 bis 30 Hz, kann aber auch außerhalb dieses Bereichs liegen. Hierbei ist zu beachten, dass die Frequenz, mit welcher die betroffenen Neuronen bei neurologischen und psychiatrischen Erkrankungen synchron feuern, üblicherweise nicht konstant ist, sondern durchaus Variationen aufweisen kann und darüber hinaus bei jedem Patienten individuelle Abweichungen zeigt.

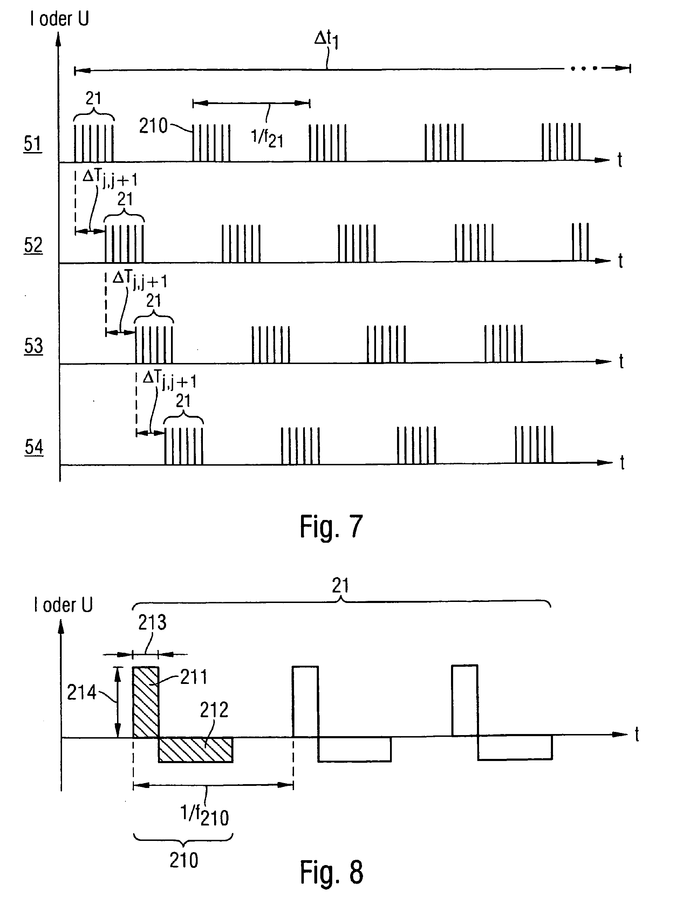

Als erste Reize 21 können beispielsweise strom- oder spannungskontrollierte Pulse verwendet werden. Ferner kann ein erster Reiz 21 ein wie in Fig. 7 dargestellter aus mehreren Einzelpulsen 210 bestehender Pulszug sein. Die Pulszüge 21 können jeweils aus 1 bis 100, insbesondere 2 bis 10, elektrischen ladungsbalancierten Einzelpulsen 210 bestehen. Die Pulszüge 21 werden z.B. als Sequenz mit bis zu 20 oder auch mehr Pulszügen appliziert. Innerhalb einer Sequenz werden die Pulszüge 21 mit der Frequenz $f_{21}$ im Bereich von 1 bis 30 Hz wiederholt.

Beispielhaft ist ein Pulszug 21, der aus drei Einzelpulsen 210 besteht, in Fig. 8 gezeigt. Die Einzelpulse 210 werden mit einer Frequenz $f_{210}$ im Bereich von 50 bis 500 Hz, insbesondere im Bereich von 100 bis 150 Hz, wiederholt. Die Einzelpulse 210 können strom- oder spannungskontrollierte Pulse sein, die sich aus einem anfänglichen Pulsanteil 211 und einem sich daran anschließenden, in entgegengesetzter Richtung fließenden Pulsanteil 212 zusammensetzen, wobei die Polarität der beiden Pulsanteile 211 und 212 gegenüber der in Fig. 8 gezeigten Polarität auch vertauscht werden kann. Die Dauer 213 des Pulsanteils 211 liegt im Bereich zwischen 1 µs und 450 µs. Die Amplitude 214 des Pulsanteils 211 liegt im Falle von stromkontrollierten Pulsen im Bereich zwischen 0 mA und 25 mA und im Fall von spannungskontrollierten Pulsen im Bereich von 0 bis 20 V. Die Amplitude des Pulsanteils 212 ist geringer als die Amplitude 214 des Pulsanteils 211. Dafür ist die Dauer des Pulsanteils 212 länger als die des Pulsanteils 211. Die Pulsanteile 211 und 212 sind idealerweise so dimensioniert, dass die Ladung, welche durch sie übertragen wird, bei beiden Pulsanteilen 211 und 212 gleich groß ist, d.h. die in Fig. 8 schraffiert eingezeichneten Flächen sind gleich groß. Im Ergebnis wird dadurch durch einen Einzelpuls 210 genauso viel Ladung in das Gewebe eingebracht, wie aus dem Gewebe entnommen wird.

Die in Fig. 8 dargestellte Rechteckform der Einzelpulse 210 stellt eine ideale Form dar. Je nach der Güte der die Einzelpulse 210 erzeugenden Elektronik wird von der idealen Rechteckform abgewichen.

Anstelle von pulsförmigen Stimulationssignalen kann die Generatoreinheit 13 beispielsweise auch anders ausgestaltete Stimulationssignale erzeugen, z.B. zeitlich kontinuierliche Reizmuster. Die oben beschriebenen Signalformen und deren Parameter sind nur beispielhaft zu verstehen. Es kann durchaus vorgesehen sein, dass von den oben angegebenen Signalformen und deren Parametern abgewichen wird.

Von dem in Fig. 6 gezeigten streng periodischen Stimulationsmuster kann auf unterschiedliche Art und Weise abgewichen werden. Beispielsweise braucht die zeitliche Verzögerung $\Delta T_{j,j+1}$ zwischen zwei aufeinander folgenden ersten Reizen 21 nicht notwendigerweise stets gleich groß zu sein. Es kann durchaus vorgesehen sein, dass die zeitlichen Abstände zwischen den einzelnen ersten Reizen 21 unterschiedlich gewählt werden. Ferner können die Verzögerungszeiten auch während der Behandlung eines Patienten variiert werden. Auch können die Verzögerungszeiten hinsichtlich der physiologischen Signallaufzeiten adjustiert werden.

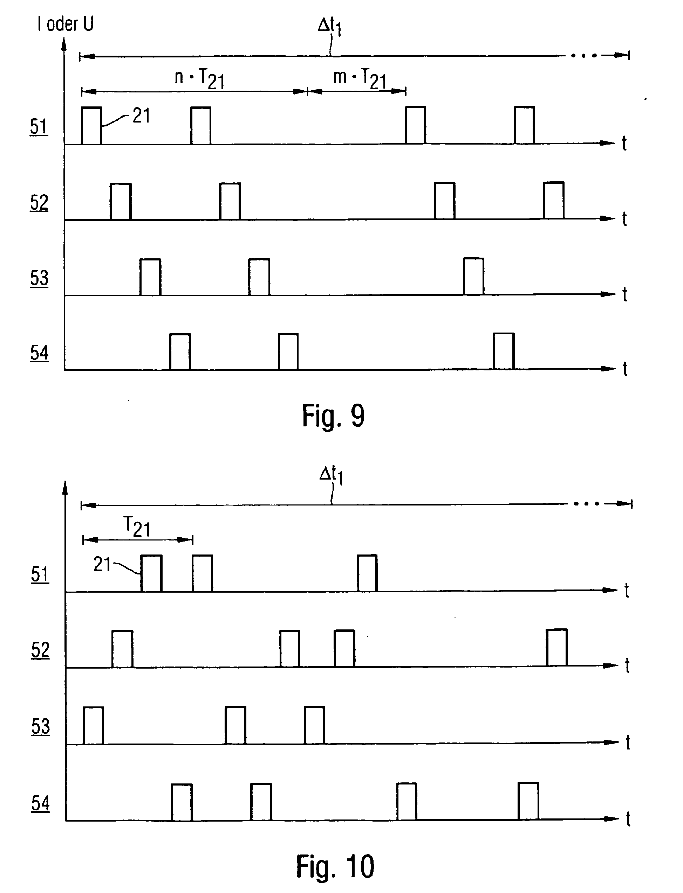

Ferner können während der Applikation der ersten Reize 21 Pausen vorgesehen werden, während derer keine Stimulation erfolgt. Eine solche Pause ist beispielhaft in Fig. 9 gezeigt. Die Pausen können beliebig lang gewählt werden und insbesondere ein ganzzahliges Vielfaches der Periode $T_{21}$ (= $1/f_{21}$) betragen. Ferner können die Pausen nach einer beliebigen Anzahl von Stimulationen eingehalten werden. Z.B. kann eine Stimulation während n aufeinander folgender Perioden der Länge $T_{21}$ durchgeführt werden und anschließend eine Pause während m Perioden der Länge $T_{21}$ ohne Stimulation eingehalten werden, wobei n und m kleine ganzen Zahlen sind, z.B. im Bereich von 1 bis 10. Dieses Schema kann entweder periodisch fortgesetzt werden oder stochastisch und/oder deterministisch, z.B. chaotisch, modifiziert werden.

Eine weitere Möglichkeit, von dem in Fig. 6 gezeigten streng periodischen Stimulationsmuster abzuweichen, besteht darin, die zeitliche Abfolge der einzelnen ersten Reize 21 stochastisch oder deterministisch oder gemischt stochastisch-deterministisch zu variieren.

Des Weiteren kann pro Periode $T_{21}$ (oder auch in anderen Zeitschritten) die Reihenfolge, in welcher die Stimulationskontaktflächen 51 bis 54 die ersten Reize 21 applizieren, variiert werden, wie dies beispielhaft in Fig. 10 gezeigt ist. Diese Variation kann stochastisch oder deterministisch oder gemischt stochastisch-deterministisch erfolgen.

Ferner kann pro Periode $T_{21}$ (oder in einem anderen Zeitintervall) nur eine bestimmte Anzahl von Stimulationskontaktflächen 51 bis 54 zur Stimulation herangezogen werden und die an der Stimulation beteiligten Stimulationskontaktflächen können in jedem Zeitintervall variiert werden. Auch diese Variation kann stochastisch oder deterministisch oder gemischt stochastisch-deterministisch erfolgen.

Alle vorstehend beschriebenen Stimulationsformen können mittels der Vorrichtung 300 auch in einem "closed loop"-Modus durchgeführt werden. Bzgl. der in Fig. 9 gezeigten Stimulationsform können der Startzeitpunkt und die Länge der Pause beispielsweise bedarfsgesteuert gewählt werden.

Wie bereits oben im Zusammenhang mit der Vorrichtung 300 beschrieben wurde, kann der "closed loop"-Modus der Vorrichtung 300 derart ausgestaltet sein, dass die von der Messeinheit 15 aufgenommenen Messsignale 26 von der Generatoreinheit 13 direkt oder gegebenenfalls nach einem oder mehreren Verarbeitungsschritten in elektrische erste Reize 21 umgesetzt werden und von der Stimulationselektrode 14 appliziert werden. In diesem Fall muss die Vorrichtung 300 nicht notwendigerweise mindestens zwei Stimulationskontaktflächen enthalten. Diese Art der Stimulation, bei welcher die am Patienten aufgenommenen Messsignale wieder in den Körper des Patienten eingespeist werden, könnte grundsätzlich auch mit nur einer einzigen Stimulationskontaktfläche durchgeführt werden, es kann jedoch auch eine beliebige, größere Anzahl von Stimulationskontaktflächen vorgesehen sein.

Der vorstehend beschriebene "closed loop"-Modus kann ebenfalls zur Desynchronisation einer Neuronenpopulation mit einer krankhaft synchronen und oszillatorischen neuronalen Aktivität eingesetzt werden.

Zur Erzeugung der ersten Reize 21 können die Messsignale 26 beispielsweise verstärkt und gegebenenfalls nach mathematischer Verrechnung (z.B. nach Mischung der Messsignale) mit einer Zeitverzögerung und linearen und/oder nicht-linearen Verrechnungsschritten als erste Reize 21 für die elektrische Stimulation verwendet werden. Der Verrechnungsmodus kann hierbei so gewählt werden, dass der krankhaften neuronalen Aktivität entgegengewirkt wird und das Stimulationssignal mit abnehmender krankhafter neuronaler Aktivität ebenfalls verschwindet oder zumindest deutlich in seiner Stärke reduziert wird.

Im Folgenden werden lineare und nicht-lineare Verarbeitungsschritte beschrieben, mit denen die mit Hilfe der Messeinheit 15 gewonnenen Messsignale 26 prozessiert werden können, bevor sie in die Stimulationselektrode 14 eingespeist werden. Bei einer nicht-linearen Verarbeitung der Messsignale 26 wird nicht die Phase der neuronalen Aktivität in den jeweiligen stimulierten Subpopulationen zurückgesetzt, sondern die Synchronisation in der krankhaft aktiven Neuronenpopulation wird unterdrückt, indem der Sättigungsprozess der Synchronisation beeinflusst wird.

Bei einer linearen Verarbeitung eines von der Messeinheit 15 gewonnenen Messsignals 26 kann das Messsignal 26 beispielsweise gefiltert und/oder verstärkt und/oder mit einer Zeitverzögerung beaufschlagt werden, bevor das so verarbeitete Signal in die Stimulationselektrode 14 eingespeist wird und von der bzw. den Stimulationskontaktflächen appliziert wird. Als Beispiel sei angenommen, dass das Messsignal 26 mittels einer EEG-Elektrode aufgenommen worden sei und die pathologische Aktivität im Zielgebiet wiedergebe. Dementsprechend ist das Messsignal 26 eine Sinusschwingung mit einer Frequenz im Bereich von 1 bis 30 Hz. Weiterhin sei beispielhaft angenommen, dass das Messsignal 26 eine Frequenz von 5 Hz aufweise. Das Messsignal 26 kann mittels eines Bandpassfilters mit einem Durchlassbereich im Bereich von 5 Hz gefiltert werden und mittels eines Verstärkers so verstärkt werden, dass es für die elektrische Hirn- oder Rückenmarkstimulation geeignete Pegel aufweist. Anschließend wird die so erhaltene verstärkte Sinusschwingung zur Ansteuerung der Stimulationselektrode 14 verwendet.

Sofern zur Stimulation eine Mehrzahl von Stimulationskontaktflächen herangezogen wird, kann das Messsignal 26 mit den in Fig. 6 gezeigten zeitlichen Verzögerungen $\Delta T_{j,j+1}$ beaufschlagt werden, bevor es als erster Reiz 21 in die entsprechenden Stimulationskontaktflächen eingespeist wird. Gemäß dem vorstehenden Beispiel werden anstelle der in Fig. 6 gezeigten rechteckförmigen Stimulationssignale 21 Sinusschwingung mit einer Frequenz von 5 Hz appliziert.

Im Folgenden wird anhand eines Beispiels erläutert, wie ein von der Messeinheit 15 gewonnenes Messsignal 26 einer nicht- linearen Prozessierung unterworfen werden kann, bevor es als erster Reiz 21 für die elektrische Hirn- oder Rückenmarkstimulation verwendet wird. Genauso wie bei der linearen Verarbeitung kann das Messsignal 26 auch hier gefiltert und/oder verstärkt und/oder mit einer Zeitverzögerung beaufschlagt werden.

Ausgangspunkt ist eine Gleichung für das Stimulationssignal S (t) (erster Reiz):

$$S(t) = K \cdot \overline{Z}^2(t) \cdot \overline{Z}^*(t - \tau) \tag{1}$$

In Gleichung (1) sind K ein Verstärkungsfaktor, der geeignet gewählt werden kann, und $\overline{Z}(t)$ eine mittlere Zustandsvariable des Messsignals. $\overline{Z}(t)$ ist eine komplexe Variable und kann folgendermaßen dargestellt werden:

$$\overline{Z}(t) = X(t) + iY(t), \tag{2}$$

wobei X(t) z.B. dem neurologischen Messsignal entsprechen kann. Da die betrachteten Frequenzen im Bereich von 10 Hz = 1/100ms = $1/T\alpha$ liegen, kann der Imaginärteil Y(t) durch X(t - $\tau\alpha$) angenähert werden, wobei beispielsweise $\tau\alpha$ = $T\alpha/4$ gilt. Damit ergibt sich:

$$S(t) = K \cdot [X(t) + iX(t - \tau_\alpha)]^2 \cdot [X(t - \tau) - iX(t - \tau - \tau_\alpha)] \tag{3}$$

Gleichung (3) kann folgendermaßen umgeformt werden:

$$\begin{aligned}
S(t) = K \cdot \big[ &X(t)^2 \cdot X(t - \tau) + i2X(t) \cdot X(t - \tau_\alpha) \cdot X(t - \tau) - X(t - \tau_\alpha) \cdot X(t - \tau) \\
&- iX(t - \tau - \tau_\alpha) \cdot X(t)^2 + 2X(t) \cdot X(t - \tau_\alpha) \cdot X(t - \tau - \tau_\alpha) \\
&+ iX(t - \tau - \tau_\alpha) \cdot X(t - \tau_\alpha) \big]
\end{aligned} \tag{4}$$

Als Stimulationssignal wird der Realteil aus Gleichung (4) verwendet:

$$real[S(t)] = K \cdot \big[ X(t)^2 \cdot X(t - \tau) - X(t - \tau_\alpha) \cdot X(t - \tau) + 2X(t) \cdot X(t - \tau_\alpha) \cdot X(t - \tau - \tau_\alpha) \big] \tag{5}$$

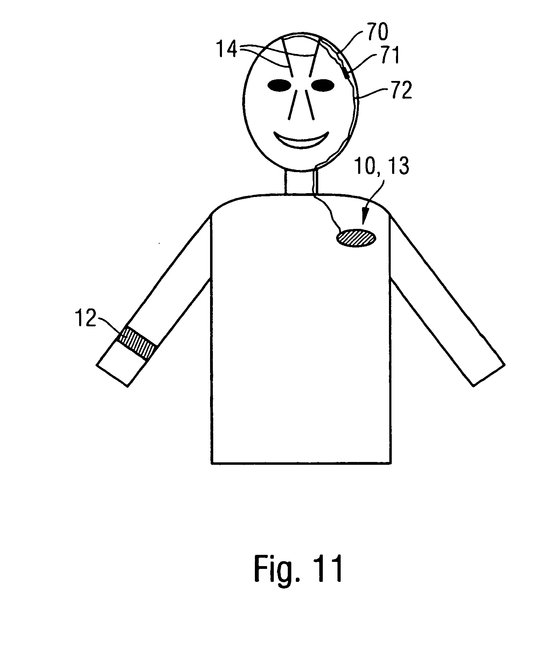

In Fig. 11 ist die Vorrichtung 100 bzw. 300 während ihres bestimmungsgemäßen Betriebs schematisch dargestellt. Dazu ist mindestens eine Stimulationselektrode 14 in einer oder beiden Seiten des Gehirns eines Patienten implantiert worden. Die in einem oder mehreren der oben genannten Zielgebiete platzierten Stimulationselektroden 14 sind jeweils mit einem Kabel 70 über einen Konnektor 71 und ein weiterführendes Kabel 72 mit der Generatoreinheit 13 verbunden. Die Generatoreinheit 13 umfasst in der vorliegenden Ausgestaltung auch die Steuereinheit 10. Die vorstehend genannten Komponenten der Vorrichtung 100 sind im Körper des Patienten implantiert. Die verbindenden Kabel 70 und 72 sowie der Konnektor 71 sind unter der Haut implantiert. Alternativ kann statt einer wie in Fig. 11 dargestellten pektoral implantierten Generatoreinheit 13 auch ein kleinerer Generator direkt im Bohrloch implantiert werden. Dadurch kann die Infektionsrate in der Generatortasche verringert werden und Brüche der verbindenden Kabel 70 und 72 vermieden werden. Ferner kann anstelle eines Vollimplantats auch ein Halbimplantat mit einer Funkverbindung verwendet werden. Im Falle einer "closed loop"-Stimulation enthält die Vorrichtung 300 noch eine Messeinheit 15. Die zweite Stimulationseinheit 12 zur Erzeugung der unspezifischen zweiten Reize 22 ist in dem vorliegenden Beispiel am Arm des Patienten angebracht. Die zweite Stimulationseinheit 12 kann mit der im Körper des Patienten befindlichen Steuereinheit 10 beispielsweise über eine Funkverbindung kommunizieren.

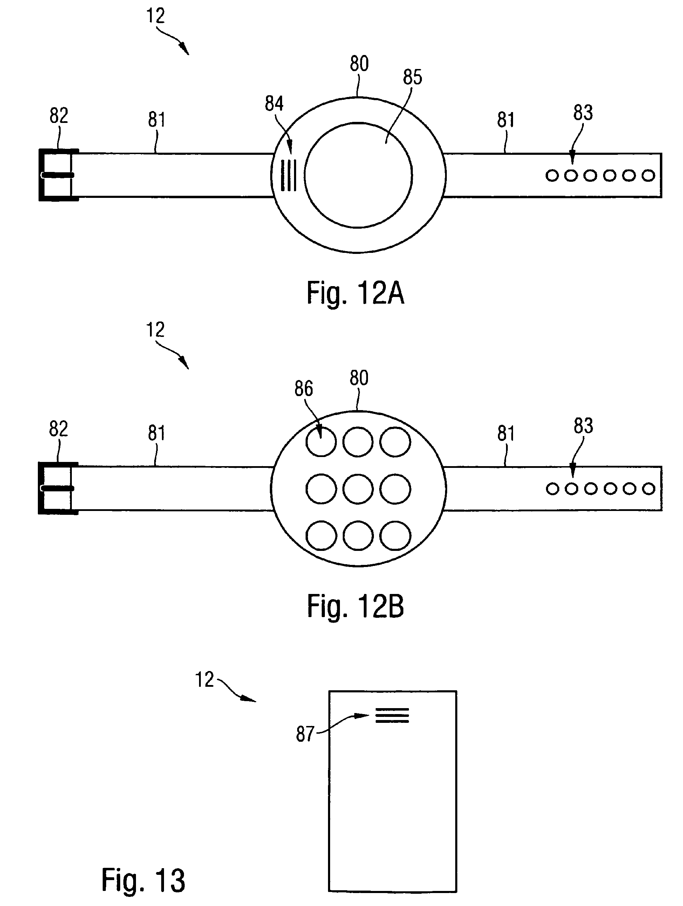

In den Fig. 12A und 12B ist ein Ausführungsbeispiel der zweiten Stimulationseinheit 12 zur Erzeugung der unspezifischen zweiten Reize 22 dargestellt. Vorliegend ist die zweite Stimulationseinheit 12 als sogenannte "Konditionierungsuhr" ausgeführt, die für den Patienten bequem zu tragen ist. In Fig. 12A ist die Vorderansicht, in Fig. 12B die Rückansicht der Konditionierungsuhr 12 gezeigt. Die Konditionierungsuhr 12 besteht aus einem Mittelteil 80, Armbändern 81, einem Verschlussteil 82 und zugehörigen Löchern 83. Alternativ kann auch ein Klettverschluss oder jeder andere gleichwertige Verschluss verwendet werden. Das Mittelteil 80 enthält einen Lautsprecher 84 zur Generierung von unspezifischen akustischen Signalen 22, z.B. einer Melodie oder einem angenehmen Summton, sowie ein Display 85 zur Generierung eines angenehmen, nicht blendenden unspezifischen optischen Reizes 22, z.B. einer sich im Wind bewegenden Blume oder einem abstrakten Lichtmuster mit hellen und warmen Farben. Ferner kann die Konditionierungsuhr 12 mit einem oder mehreren Vibratoren 86 ausgestattet sein, die unspezifische taktile und/oder vibratorische Reize 22 generieren. Zur Erzeugung taktiler Reize 22 können die Vibratoren mit Frequenzen von kleiner 1 Hz betrieben werden. Insbesondere können die beweglichen Teile der Vibratoren 86 in diesem Fall so ausgerichtet sein, dass sie Druckreize besser realisieren können, d.h. die Hauptbewegungsrichtung der Vibratoren 86 sollte in die Haut hinein gerichtet sein. Ferner könnten die taktilen Reize 22 auch durch Druckaktuatoren oder sich langsam relativ zur Haut bewegende Elemente, die z.B. in die Konditionierungsuhr integriert sein können, erzeugt werden. Sofern mittels der Vibratoren 86 vibratorische Reize 22 erzeugt werden sollen, können Vibrationsfrequenzen im Bereich von 10 bis 160 Hz oder darüber eingesetzt werden. In diesem Fall können die beweglichen Teile der Vibratoren 86 eine ausgeprägte Bewegungsrichtung im Wesentlichen parallel zur Haut haben. Die Vibratoren 86 können auch so betrieben werden, dass sie gleichzeitig taktile und vibratorische Reize 22 erzeugen.

Die Konditionierungsuhr 12 kann auch so ausgestaltet sein, dass sie nur einen unspezifischen Reiz 22 einer Sinnesmodalität, z.B. nur einen optischen Reiz, generiert. Die Stromversorgung der Konditionierungsuhr 12 erfolgt durch eine Batterie und/oder Solarzellen und/oder ein mechanisches Schwungrad im Inneren der Konditionierungsuhr 12.

Zur Kontrolle des Stimulationseffekts kann die Konditionierungsuhr 12 zusätzlich ein Akzelerometer beinhalten, mit dem sich die krankhafte oszillatorische Aktivität, z.B. von krankhaftem Tremor, oder aber das mittlere Aktivitätsniveau des Patienten messen lässt. Das mittlere Aktivitätsniveau des Patienten spiegelt die Verlangsamung bzw. Verarmung der Bewegungen bzw. der Bewegungsunfähigkeit des Patienten wider (d.h. die Brady-, Hypo- und Akinese).

Ein weiteres Ausführungsbeispiel der zweiten Stimulationseinheit 12 ist Fig. 13 schematisch dargestellt. Dabei handelt es sich um einen beispielsweise handyförmigen Stimulator, der z.B. in der Hemdtasche oder Hosentasche des Patienten getragen werden kann und der mittels eines Lautsprechers 87 unspezifische akustische Reize 22 generiert.

Ferner kann ein externes Programmiergerät für den Arzt vorgesehen sein, mit welchem die Parameter der Steuereinheit 10, der Generatoreinheit 13 und/oder der unspezifischen, physiologischen Stimulationseinheit 12 eingestellt werden können. Darüber hinaus kann dem Patienten ebenfalls ein externes Programmiergerät zur Verfügung gestellt werden, mit welchem der Patient die Stimulationsgeräte ausstellen kann bzw. Parameter der Stimulationseinheiten 11 und 12 in engen, vom Arzt vorgegebenen Grenzen modifizieren kann. Ferner kann das für den Patienten bestimmte Programmiergerät die weiter oben bereits beschriebene Funktionalität enthalten, mittels welcher der Patient selbstständig, z.B. durch das Betätigen einer Taste, ein Umschalten vom zweiten Betriebsmodus in den ersten Betriebsmodus, also die Lernphase, bewirken kann, wenn er sich nicht ausreichend therapiert fühlt, wenn also z.B. sein Tremor oder seine Unbeweglichkeit zu stark sind. Die Programmiergeräte können beispielsweise über Funkverbindungen mit den jeweiligen Komponenten des Stimulationsgeräts kommunizieren.

**Patentansprüche**

1. Vorrichtung (100; 300) umfassend:

- eine erste Stimulationseinheit (11) zur Erzeugung von elektrischen ersten Reizen (21), die bei einer Verabreichung an das Gehirn und/oder Rückenmark eines Patienten eine krankhaft synchrone Aktivität von Neuronen im Gehirn und/oder Rückenmark des Patienten unterdrücken, **gekennzeichnet durch**:
- eine zweite Stimulationseinheit (12) zur Erzeugung von optischen und/oder akustischen und/oder taktilen und/oder vibratorischen zweiten Reizen (22), und
- eine Steuereinheit (10) zur Steuerung der ersten und zweiten Stimulationseinheit (11, 12), wobei
- die Erzeugung der ersten und zweiten Reize (21, 22) wahlweise in einem ersten oder einem zweiten Betriebsmodus erfolgt, und
- die Steuereinheit (10) geeignet ist die erste und zweite Stimulationseinheit (11, 12) derart anzusteuern, dass

**EP 2 358 430 B1**

im ersten Betriebsmodus die Erzeugung von mindestens 60% der zweiten Reize (22) zeitlich an die Erzeugung der ersten Reize (21) gekoppelt ist und im zweiten Betriebsmodus die Erzeugung von mindestens 60% der zweiten Reize (22) ohne die Erzeugung der ersten Reize (21) erfolgt.

2. Vorrichtung (100; 300) nach Anspruch 1, wobei im ersten Betriebsmodus die Erzeugung von mindestens 80% der zweiten Reize (22) zeitlich an die Erzeugung der ersten Reize (21) gekoppelt ist und/oder im zweiten Betriebsmodus die Erzeugung von mindestens 80% der zweiten Reize (22) ohne die Erzeugung der ersten Reize (21) erfolgt.

3. Vorrichtung (100; 300) nach Anspruch 1 oder 2, wobei die erste Stimulationseinheit (11) mindestens eine Elektrode (14) zur Verabreichung der ersten Reize (21) an das Gehirn und/oder Rückenmark des Patienten umfasst.

4. Vorrichtung (300) nach einem der vorhergehenden Ansprüche, wobei die Vorrichtung (300) eine Messeinheit (15) zum Aufnehmen von Messsignalen (26) umfasst, welche die krankhaft synchrone Aktivität der Neuronen wiedergeben.

5. Vorrichtung (300) nach Anspruch 4, wobei die Steuereinheit geeignet ist (10) im zweiten Betriebsmodus die Zahl der ersten Reize (21), deren Erzeugung zeitlich an die Erzeugung der zweiten Reize (22) gekoppelt ist zu erhöhn, falls die Messsignale (26) einen vorgegebenen ersten Schwellwert überschreiten.

6. Vorrichtung (300) nach Anspruch 4 oder 5, wobei die Steuereinheit (10) geeignet ist von dem zweiten Betriebsmodus in den ersten Betriebsmodus zu wechseln, falls die Messsignale (26) einen vorgegebenen zweiten Schwellwert überschreiten.

7. Vorrichtung (300) nach den Ansprüchen 5 und 6, wobei der erste Schwellwert kleiner als der zweite Schwellwert ist.

8. Vorrichtung (300) nach einem der Ansprüche 4 bis 7, wobei die erste Stimulationseinheit (11) geeignet ist die Messsignale (26) als erste Reize (21) einzusetzn oder die Messsignale (26) nach einer Weiterverarbeitung als erste Reize (21) einzusetzn.

9. Vorrichtung (100; 300) nach einem der vorhergehenden Ansprüche, geeignet, um die ersten Reize (21) in ersten Zeitabschnitten ($\Delta t_1$) zu erzeugn und die zweiten Reize (22) in zweiten Zeitabschnitten ($\Delta t_2$) zu erzeugn wobei im ersten Betriebsmodus mindestens 60% der zweiten Zeitabschnitte ($\Delta t_2$) jeweils mit einem der ersten Zeitabschnitte ($\Delta t_1$) zeitlich überlappen.

10. Vorrichtung (100; 300) nach Anspruch 9, wobei der zeitliche Überlapp ($\Delta t_{12}$) eines der ersten Zeitabschnitte ($\Delta t_1$) mit einem der zweiten Zeitabschnitte ($\Delta t_2$) mindestens 10% der Zeitdauer des zweiten Zeitabschnitts ($\Delta t_2$) beträgt.

11. Vorrichtung (100; 300) nach einem der vorhergehenden Ansprüche, wobei die ersten Reize (21) jeweils Sequenzen von elektrischen Pulsen (210) sind.

12. Vorrichtung (100; 300) nach einem der vorhergehenden Ansprüche, wobei die Vorrichtung (100; 300) ein Programmiergerät umfasst, mittels welchem der Patient ein Wechsel von dem zweiten Betriebsmodus in den ersten Betriebsmodus bewirken kann.

**Claims**

1. An apparatus (100; 300) comprising:

- a first stimulation unit (11) for generating electrical first stimuli (21) which suppress a pathologically synchronous activity of neurons in the brain and/or spinal cord of a patient when administered to the brain and/or spinal cord of the patient, **characterised by**
- a second stimulation unit (12) for generating optical and/or acoustic and/or tactile and/or vibratory second stimuli (22); and
- a control unit (10) for controlling the first and second stimulation units (11, 12), wherein
- the generation of the first and second stimuli (21, 22) selectively takes place in a first or in a second operating mode; and
- the control unit (10) is suitable to control the first and second stimulation units (11, 12) such that the generation of at least 60% of the second stimuli (22) is coupled in time to the generation of the first stimuli (21) in the first operating mode and the generation of at least 60% of the second stimuli (22) takes place without the generation of the first stimuli (21) in the second operating mode.

2. An apparatus (100; 300) in accordance with claim 1, wherein the generation of at least 80% of the second stimuli (22) is coupled in time to the generation of the first stimuli (21) in the first operating mode and/or the generation of at least 80% of the second stimuli (22) takes place without the generation of the first stimuli (21) in the second operating mode.

3. An apparatus (100; 300) in accordance with claim 1 or claim 2, wherein the first stimulation unit (11) includes at least one electrode (14) for administering the first stimuli (21) to the brain and/or spinal cord of the patient.

4. An apparatus (300) in accordance with any one of the preceding claims, wherein the apparatus (300) includes a measuring unit (15) for picking up measured signals (26) which represent the pathologically synchronous activity of the neurons.

5. An apparatus (300) in accordance with claim 4, wherein the control unit (10) is suitable to increase the number of first stimuli (21) whose generation is coupled in time to the generation of the second stimuli (22) in the second operating mode if the measured signals (26) exceed a predefined first threshold value.

6. An apparatus (300) in accordance with claim 4 or claim 5, wherein the control unit (10) is suitable to change from the second operating mode into the first operating mode if the measured signals (26) exceed a predefined second threshold value.

7. An apparatus (300) in accordance with claims 5 and 6, wherein the first threshold value is smaller than the second threshold value.

8. An apparatus (300) in accordance with any one of the claims 4 to 7, wherein the first stimulation unit (11) is suitable to use the measured signals (26) as first stimuli (21) or to use the measured signals (26) as first stimuli (21) after a further processing.

9. An apparatus (100; 300) in accordance with any one of the preceding claims which is suitable to generate the first stimuli (21) in first time periods ($\Delta t_1$) and to generate the second stimuli (22) in second time periods ($\Delta t_2$), wherein at least 60% of the second time periods ($\Delta t_2$) respectively overlap with one of the first time periods ($\Delta t_1$) in the first operating mode.

10. An apparatus (100; 300) in accordance with claim 9, wherein the time overlap ($\Delta t_{12}$) of one of the first time periods ($\Delta t_1$) with one of the second time periods ($\Delta t_2$) amounts to at least 10% of the time duration of the second time period ($\Delta t_2$).

11. An apparatus (100; 300) in accordance with any one of the preceding claims, wherein the first stimuli (21) are each sequences of electrical pulses (210).

12. An apparatus (100; 300) in accordance with any one of the preceding claims, wherein the apparatus (100; 300) includes a programing unit, by means of which the patient can effect a change from the second operating mode into the first operating mode.

**Revendications**

1. Dispositif (100 ; 300) comprenant :

   - une première unité de stimulation (11) pour engendrer des premières excitations électriques (21) qui, lors de l'application au cerveau et/ou à la moelle épinière d'un patient, bloquent une activité synchrone pathologique de neurones dans le cerveau et/ou dans la moelle épinière du patient,

   **caractérisé par** :

- une seconde unité de stimulation (12) pour engendrer des secondes excitations (22) optiques et/ou acoustiques et/ou tactiles et/ou vibratoires, et

- une unité de commande (10) pour commander la première et la seconde unité de stimulation (11, 12), dans lequel

- la génération des premières et des secondes excitations (21, 22) a lieu sélectivement dans un premier ou dans un second mode de fonctionnement, et

- l'unité de commande (10) est appropriée pour piloter la première et la seconde unité de stimulation (11, 12) de telle façon que dans le premier mode de fonctionnement la génération d'au moins 60 % des secondes excitations (22) est couplée temporellement à la génération des premières excitations (21) et que dans le second mode de fonctionnement la génération d'au moins 60 % des secondes excitations (22) a lieu sans la génération des premières excitations (21).

2. Dispositif (100 ; 300) selon la revendication 1, dans lequel dans le premier mode de fonctionnement, la génération d'au moins 80 % des secondes excitations (22) est couplée temporellement à la génération des premières excitations (21), et/ou dans le second mode de fonctionnement la génération d'au moins 80 % des secondes excitations (22) a lieu sans la génération des premières excitations (21).

3. Dispositif (100 ; 300) selon la revendication 1 ou 2, dans lequel la première unité de stimulation (11) comprend au moins une électrode (14) pour l'application des premières excitations (21) au cerveau et/ou à la moelle épinière du patient.

4. Dispositif (300) selon l'une des revendications précédentes, dans lequel le dispositif (300) comprend une unité de mesure (15) pour enregistrer des signaux de mesure (26) qui reproduisent l'activité synchrone pathologique des neurones.

5. Dispositif (300) selon la revendication 4, dans lequel l'unité de commande (10) est appropriée pour augmenter, dans le second mode de fonctionnement, le nombre des premières excitations (21) dont la génération est temporellement couplée à la génération des secondes excitations (22) au cas où les signaux de mesure (26) dépassent une première valeur seuil prédéterminée.

6. Dispositif (300) selon la revendication 4 ou 5, dans lequel l'unité de commande (10) est appropriée pour changer depuis le second mode de fonctionnement vers le premier mode de fonctionnement au cas où les signaux de mesure (26) dépassent une seconde valeur seuil prédéterminée.

7. Dispositif (300) selon les revendications 5 et 6, dans lequel la première valeur seuil est plus petite que la seconde valeur seuil.

8. Dispositif (300) selon l'une des revendications 4 à 7, dans lequel la première unité de stimulation (11) est appropriée pour employer les signaux de mesure (26) comme premières excitations (21) ou pour employer les signaux de mesure (26) comme premières excitations (21) après un post-traitement.

9. Dispositif (100 ; 300) selon l'une des revendications précédentes, approprié pour générer les premières excitations (21) dans des premières fenêtres temporelles ($\Delta t_1$) et pour générer les secondes excitations (22) dans des secondes fenêtres temporelles ($\Delta t_2$), et dans le premier mode de fonctionnement au moins 60 % des secondes fenêtres temporelles ($\Delta t_2$) chevauchent temporellement respectivement une des premières fenêtres temporelles ($\Delta t_1$).

10. Dispositif (100 ; 300) selon la revendication 9, dans lequel le chevauchement temporel ($\Delta t_{12}$) de l'une des premières fenêtres temporelles ($\Delta t_1$) avec l'une des secondes fenêtres temporelles ($\Delta t_2$) s'élève à au moins 10 % de la durée de la seconde fenêtre temporelle ($\Delta t_2$).

11. Dispositif (100 ; 300) selon l'une des revendications précédentes, dans lequel les premières excitations (21) sont respectivement des séquences d'impulsions électriques (210)

12. Dispositif (100 ; 300) selon l'une des revendications précédentes, dans lequel le dispositif (100 ; 300) inclut un appareil de programmation au moyen duquel le patient peuvent provoquer un changement depuis le second mode de fonctionnement vers le premier mode de fonctionnement.

Fig. 1

Fig. 2A

Fig. 2B

Fig. 3

Fig. 4

Fig. 5

Fig. 6

Fig. 7

Fig. 8

Fig. 9

Fig. 10

Fig. 11

12

84 80 85

82 81 81 83

Fig. 12A

12

86 80

82 81 81 83

Fig. 12B

12

87

Fig. 13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1944059 A2
- DE 10233960 A1